# EUROPEAN PATENT SPECIFICATION

(11) **EP 2 364 673 B1**
(45) Date of publication and mention of the grant of the patent: **02.12.2015**
(21) Application number: 11156977.8
(22) Date of filing: 04.03.2011
(51) Int. Cl.: A61F 2/16

(54) **Intraocular lens injection instrument**
Injektionsvorrichtung für eine Intraokularlinse
Instrument d'injection de lentille intraoculaire

(30) Priority: 09.03.2010 JP 2010052359; 09.03.2010 JP 2010052360; 15.11.2010 JP 2010255289
(43) Date of publication of application: 14.09.2011
(73) Proprietor: Nidek Co., Ltd., Gamagori-shi, Aichi 443-0038 (JP)
(72) Inventor: Nagasaka, Shinji, Gamagori-shi Aichi 443-0038 (JP); Natsume, Akiyoshi, Gamagori-shi Aichi 443-0038 (JP); Hisada, Shota, Gamagori-shi Aichi 443-0038 (JP); Kobayashi, Takashi, Gamagori-shi Aichi 443-0038 (JP)
(74) Representative: Prüfer & Partner GbR European Patent Attorneys

(56) References cited:
- EP-A1- 2 085 053
- EP-A1- 2 255 751
- WO-A1-2010/080351

## Description

### Technical Field

The present invention relates to an IOL injection instrument for injecting an intraocular lens into an eye.

### Background Art

As one of operative treatment instruments for cataract, heretofore, there has generally been used an intraocular lens injection instrument (hereinafter, referred to as an injector) for injecting an intraocular lens (IOL) into a patient's eye from which a clouded lens has been removed. Such injector has a tapered front end. Accordingly, when the IOL placed in the injector is pushed toward a front end of the injector by use of a push rod called a plunger, the IOL is folded into a smaller size along the shape of an inner wall of the injector. Since the IOL in a small folded state is to be injected in the eye, an incision to be formed in the eye can be made as small in diameter as possible. This reduces a burden on a patient and prevents the generation of aftereffect such as postoperative astigmatism (EP1728488A1, JP 2006-333924A).

In such injector, an optical part to be folded in an injection part (an injection tubular part) is made smaller in diameter as moving toward the front end of the tapered injection part. Thus, the optical part becomes strongly pushed by the plunger. And, if the optical part is pushed by the plunger even though the optical part is not sufficiently folded in the injection part, the optical part made of a soft material is deformed. And then the front end of the plunger slightly gets onto the optical part, accordingly, this may cause a part of the optical part to enter a gap between the front end and the inner wall surface of the injection part. When the optical part is further pushed from the above state by the plunger, the part of the optical part is liable to further slip into the gap. Accordingly, the optical part may be broken or allow the plunger to further get onto the optical part.

A front opening of the injector is formed as small as possible in order to minimize the incision to be made in the eye. Accordingly, if a rear support part is caught between the front opening of the injector and the plunger during push-out of the IOL, the support part may be broken by a push-out motion of the IOL. Further, when the rear support part slides on the surface of the plunger or the like during push-out of the IOL using the injector, the rear support part may spread backward inside the injection part (backward in a forward moving direction of the IOL). In association with the push-out motion of the injector by the plunger, therefore, the rear support part entwines the plunger and others and hence broken.

EP-A-2 085 053 discloses an IOL-injection instrument provided with a plunger. According to said document, there is disclosed a technique of adjusting operating pressure (a pushing touch) in pushing a plunger into an IOL to a constant level. The operating pressure is adjusted by means of a protrusion in the main body of the injection instrument which contacts a part of the plunger when ejecting an IOL.

### Summary of Invention

The present invention has been made to solve the above problems and has a purpose to provide an IOL injection instrument capable of appropriately pushing an IOL into an eye.

### Solution to Problem

To achieve the above purpose, one aspect of the invention provides an intraocular lens injection instrument for injecting an intraocular lens (IOL) according to claim 1.

Further developments of the present invention are given in the dependent claims.

### Brief Description of Drawings

FIG. 1 is an explanatory view showing a configuration of a one-piece intraocular lens;
FIGs. 2A and 2B are explanatory views showing a configuration of a three-piece IOL;
FIGs. 3A and 3B are explanatory views showing an external configuration of an injector;
FIGs. 4A and 4B are explanatory views showing a configuration of a plunger;
FIG. 5 is a schematic cross sectional view showing an internal structure of the injector;
FIGs. 6A to 6D are explanatory views showing a relationship between a front end and an injection part viewed from front in a cross-section perpendicular to a push-out direction;
FIG. 7 is an enlarge view of the front end viewed from side;
FIGs. 8A to 8D are schematic cross-sectional views of an interior of the injection part and its surroundings viewed from side;
FIGs. 9A and 9D are explanatory views of modified patterns of asperities;
FIGs. 10A and 10B are explanatory views showing a configuration of a plunger in a second embodiment;
FIGs. 11A and 11B are enlarged views of a front end of the plunger and its surroundings;
FIGs. 12A to 12C are views showing a modified example of the plunger in the second embodiment;
FIGs. 13A to 13C are views showing another modified example of the plunger in the second embodiment;
FIGs. 14A to 14C are explanatory views showing a push-out motion using the plunger in the second embodiment;
FIG. 15 is a view showing another modified example of the plunger in the second embodiment; and
FIG. 16 is a view showing another modified example of the plunger in the second embodiment.

### Description of Embodiments

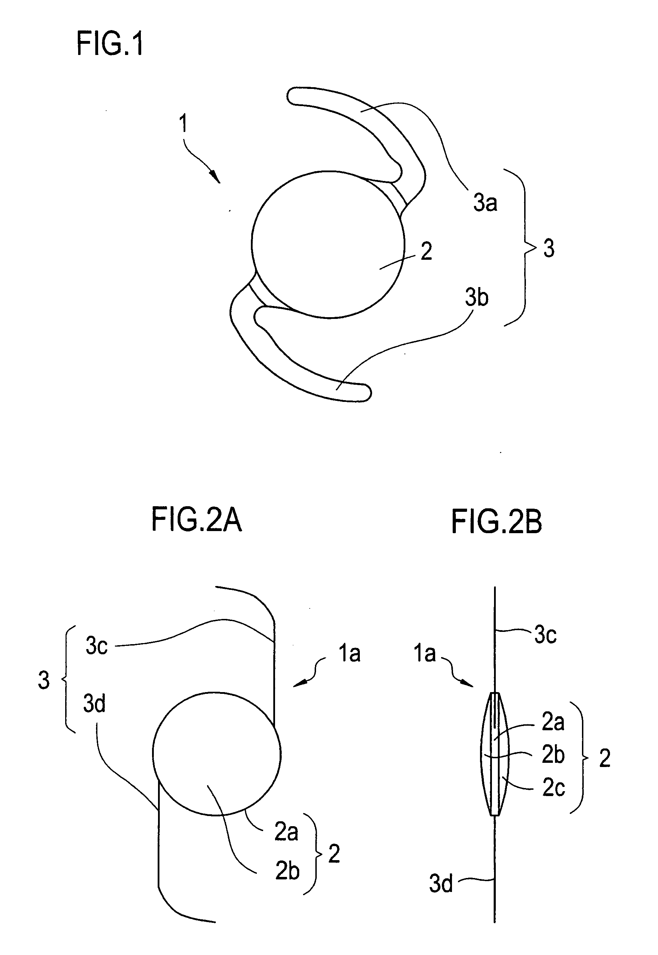

A detailed description of a preferred embodiment of the present invention will now be given referring to the accompanying drawings. FIG. 1 is an explanatory view showing a structure of an intraocular lens (IOL) 1 of one-piece type. The IOL 1 includes an optical part 2 having a predetermined refractive power and a pair of support parts 3 (a front support part 3a and a rear support part 3b arranged front and back in a push-out direction of the IOL) for supporting the optical part 2 in an eye. The IOL 1 is integrally made of a foldable soft material such as a monomeric material such as HEMA (hydroxyethyl methacrylate) and a composite material of acrylic ester and methacrylate ester by cutting, molding, or another technique.

FIGs. 2A and 2B are explanatory views showing a structure of an IOL 1a of a three-piece type; specifically, FIG. 2A is a front view of the IOL 1a and FIG. 2B is a side view of the same. The three-piece IOL 1a is made in such a way that an optical part 2 and loop-shaped (C-shaped, J-shaped) support parts 3 each having a free end (a front support part 3a and a rear support part 3b arranged front and back in a push-out direction) are separately produced and then combined in an integral form.

The optical part 2 has predetermined refractive power and includes a peripheral surface (hereinafter, referred to as an "edge") 2a to which a plunger 40 comes into contact when the IOL 1a is to be pushed out of the IOL injection instrument (hereinafter, referred to as an "injector") 10 mentioned later, a front surface 2b to be positioned on a cornea side in the eye, and a rear surface 2c to be positioned on a retina side. The optical part 2 is made of a material conventionally used for a foldable soft IOL, e.g., a monomeric material such as HEMA (hydroxyethyl methacrylate), a composite material of acrylic ester and methacrylate ester, and others. Further, the support parts 3 are made of a material conventionally used for IOL's support parts such as PMMA (polymethacrylate).

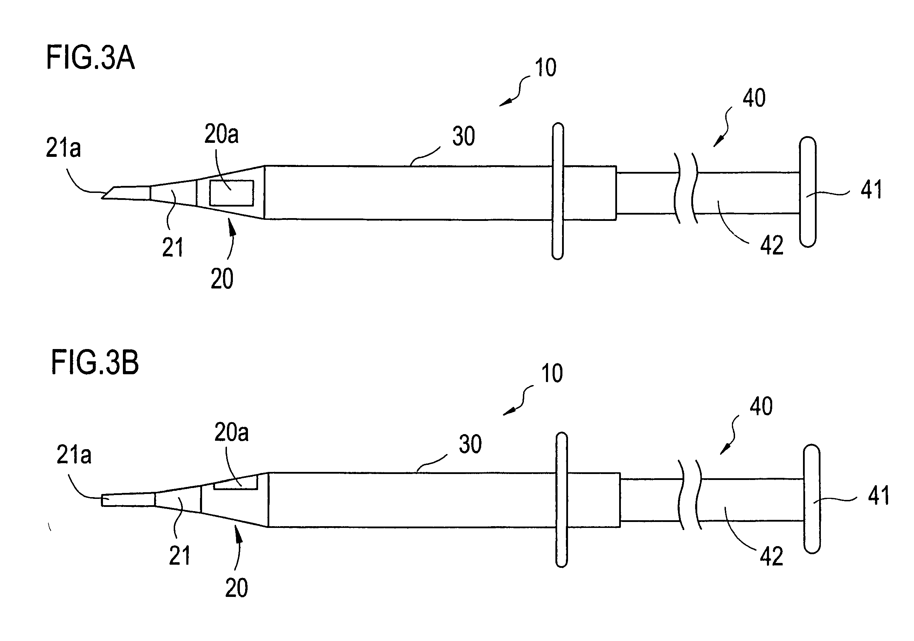

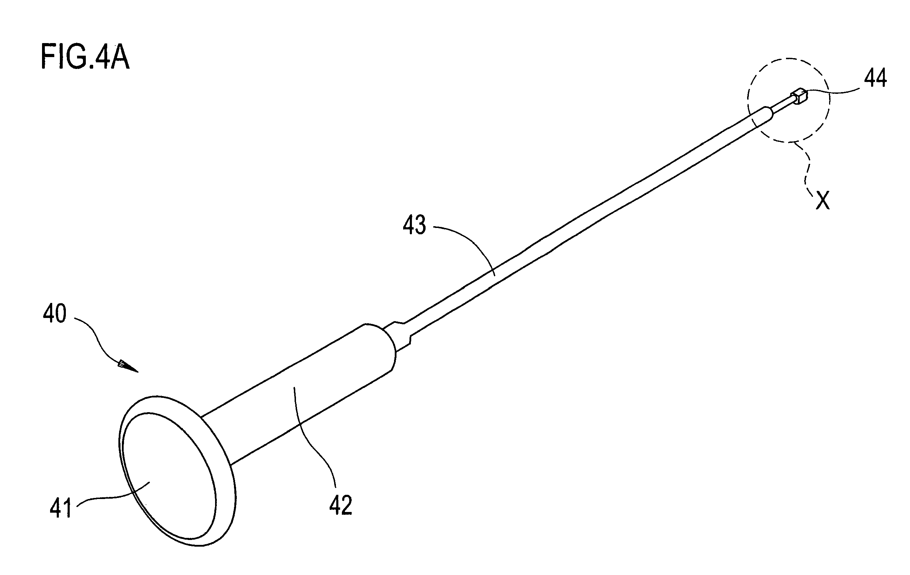

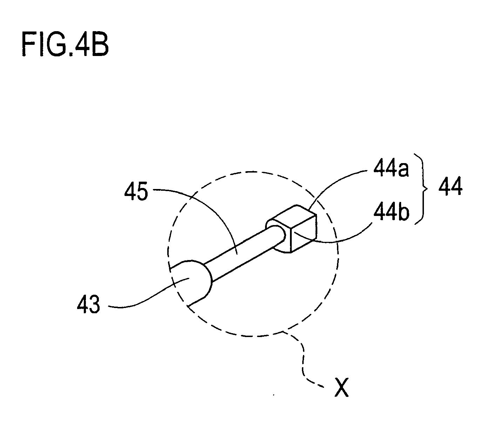

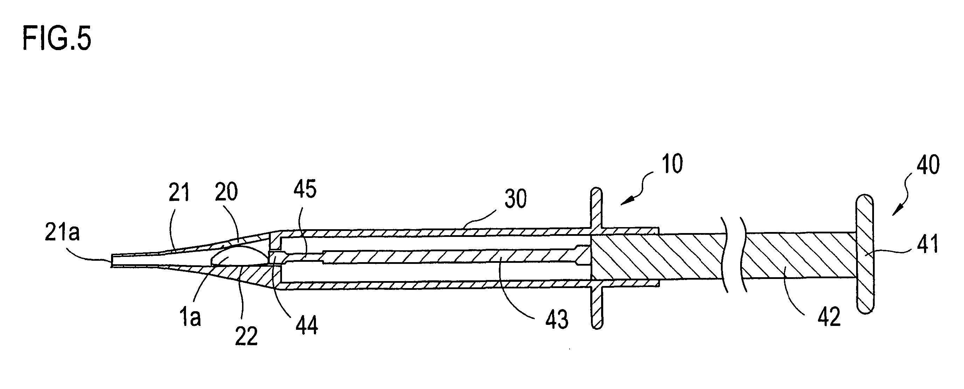

FIGs. 3A and 3B are schematic external views of the injector 10. Specifically, FIG. 3A is a top view of the injector 10 and FIG. 3B is a side view of the same. FIGs. 4A and 4B are explanatory views showing a structure of a push-out member (hereinafter, referred to as a "plunger") 40; specifically, FIG. 4 is a perspective view of the plunger 40 and FIG. 4B is an enlarged view of a part X in FIG. 4A. FIG. 5 is a schematic cross-sectional view showing an internal structure of the plunger 10. The plunger 10 includes a hollow injection tubular part (hereinafter, an injection part) 20 for folding the IOL 1 into a smaller form, a hollow cylindrical body part 30 (hereinafter, referred to as a "body part") provided at a front end with the injection part 20, and the plunger 40 for pushing out the IOL 1. It is to be noted that the plunger 40 is attached to the body part 30 so as to be movable back and forth.

The injection part 20 includes an injection tube 21 having a front end 21 a, and a lid member 20a. The IOL 1 is put on a setting part (a setting stage) 22 inside the injection tube 21 through an opening of the lid member 20a. On the other hand, the injection tube 21 is formed in a tapered shape having a region whose inner diameter is gradually smaller (thinner) toward the frond end 21a. The IOL 1 is folded into a smaller size by passing the interior of the injection tube 21 and then injected out of the front end 21 a.

The details of the plunger 40 in this embodiment will be explained below. The plunger 40 includes a press part 41 to be pressed by an operator, an axial base part 42 joined with the press part 41, and a push rod 43 connected to the axial base part 42, a tip portion 44 including a contact surface 44a which can come into contact with the optical part 2 during push-out operation of the IOL 1, and a shaft 45 joining the push rod 43 and the tip portion 44.

The contact surface 44a is formed as a flat plane that is vertical to the push-out axis of the plunger 40 and that has an area able to pass through the front end 21a together with the rear support part 3b (or 3d). Thus, the contact surface 44a can be appropriately brought into contact with the optical part 2. The shaft 45 has a smaller diameter than the diameters of the push rod 43 and the tip portion 44. Accordingly, an escape portion is created between the inner wall of the front end 21a of the injection part 20 and the tip portion 44 of the plunger 40 to receive the rear support part 3b (or 3d) during push-out of the IOL 1. This configuration prevents the support part 3b (or 3d) from becoming caught and broken and other defects. The thus configured plunger 40 is inserted to be movable back and forth in a passage continuously extending through the body part 30 to the front end 21a of the injection part 20. A part of an outer peripheral region of the tip portion 44 (the peripheral surface 44b) of the plunger 40 in the present embodiment is formed to have an outer shape along the inner wall of the injection tube 21. The outer shape (surface) is formed, in a predetermined range thereof, with asperities or irregulalities (projections and recesses) to prevent a part of the optical part 2 from getting into between the tip portion 44 and the inner wall of the injection tube 21.

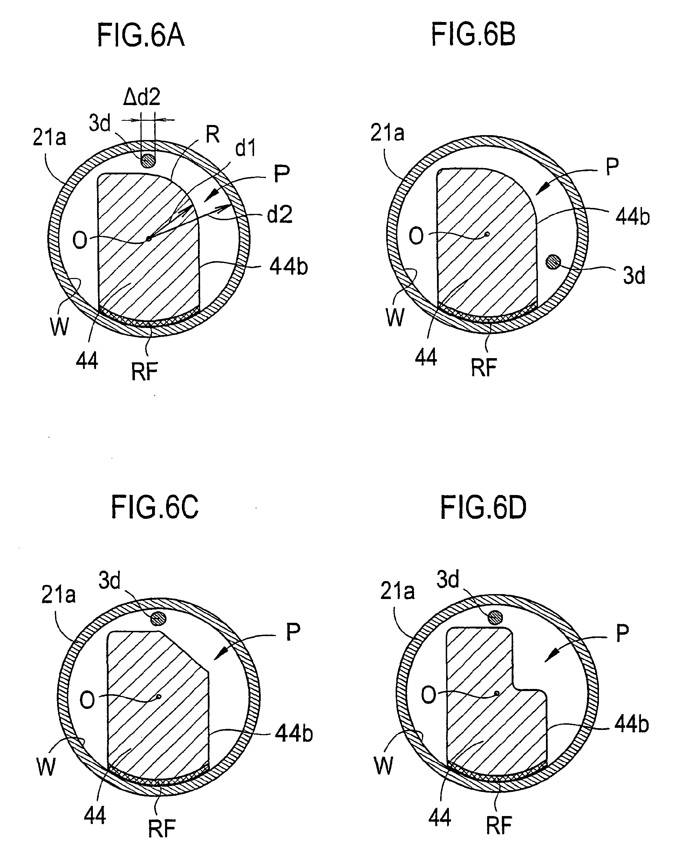

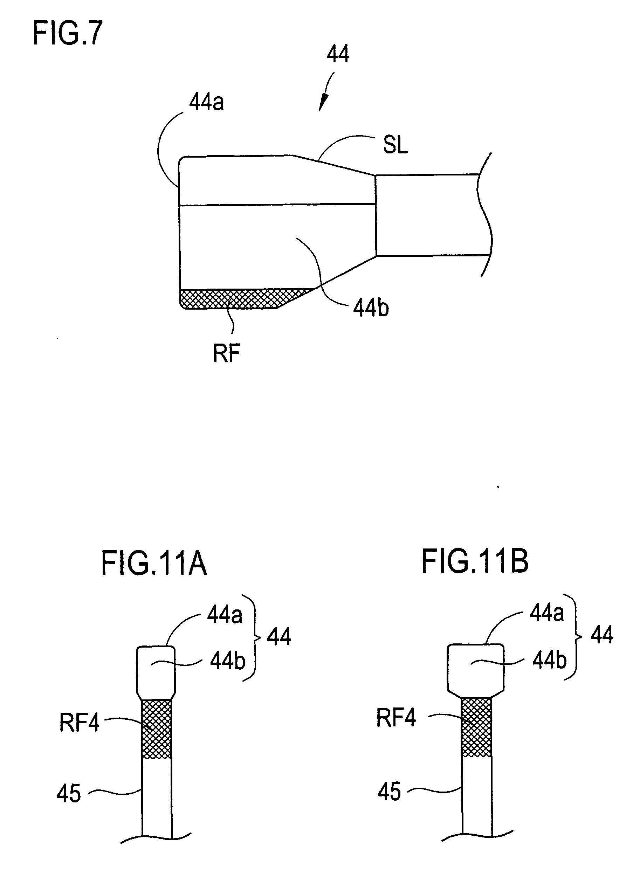

The shape of the tip portion 44 will be described in detail below. The following explanation is given to an example using the three-piece IOL 1a. FIGs. 6A to 6D are explanatory views showing a positional relationship between the tip portion 44 and the rear support part 3d when viewed from front in a cross-section perpendicular to the push-out direction while the tip portion 44 is placed near the front end 21a of the injection tube 21. Specifically, FIG. 6A shows a positional relationship between the tip portion 44 and the rear support part 3d while the optical part 2 is in a folded state in the injection part 20 and FIG. 6B shows a positional relationship between the tip portion 44 and the rear support part 3d while the optical part 2 is in an open state in the eye. Further, FIG. 7 is an enlarged view of the tip portion 44 seen from side.

The tip portion 44 is designed to have a size (a diameter) allowed to pass through the inner passage of the injection part 20 and make the contact surface 44a sufficiently come into contact with the edge 2a of the optical part 2 in order to inject the IOL 1a out of the injection part 20. The tip portion 44 also has an outer shape (the peripheral surface 44b) including a predetermined region (a lower surface) shaped along an inner wall surface W of the injection part 20 and a length in the push-out direction enough to move ahead while rubbing the inner wall surface W. Accordingly, the tip portion 44 (the lower surface of the peripheral surface 44b) is moved with no gap with respect to the inner wall surface W. This prevents the tip portion 44 from biting or getting onto the optical part 2 and thus damaging the optical part 2. In this case, preferably, the push-out axis of the plunger 40 is formed to slightly incline downward so that the lower surface of the tip portion 44 (the peripheral surface 44b) is pressed against the lower surface of the inner wall surface W. This configuration makes it easier to prevent the tip portion 44 from getting onto the optical part 2.

In the present embodiment, furthermore, a part of the outer shape (the peripheral surface 44b) of the tip portion 44 viewed in a cross-section perpendicular to the push-out direction is formed as a predetermined curved surface R.
In the injector 10, the front end 21a of the injection part 20 is designed to be as small as possible in order to minimize an incision to be formed in the patient's eye. On the other hand, if the opening diameter of the front end 21 a is small, the rear support part 3d is liable to be caught between the plunger 40 and the opening of the front end 21a of the injection part 20 when the IOL 1a is being pushed out. If the rear support part 3d is left in the injection tube 21 after the optical part 2 is first pushed out into the eye, and the rear support part 3d gets caught between the tip portion 44 of the plunger 40 and the inner wall surface W of the injection tube 21, the optical part 2 is likely to open by centering around the rear support part 3d. If the optical part 2 opens by centering around the rear support part 3d, the optical part 2 comes to an inclined (standing) state in the eye. In this case, the operator has to adjust the optical part 2 to a proper position in the eye. This takes a lot of time. On the other hand, a longer surgical time is also a burden on the patient.

In the present embodiment, the predetermined range (a part) of the outer periphery (the peripheral surface 44b) of the tip portion 44 is formed as a curved surface R. Hereby the rear support part 3d is moved in the injection part 20 while the optical part 2 is being folded in the injection part 20 and the folded optical part 2 is being opened in the eye. This configuration allows the rear support part 3d located near the front end 21a of the injection part 20 and above the peripheral surface 44b of the tip portion 44 to pass through a gap (a clearance) P mentioned later in association with subsequent behaviors (opening) of the optical part 2 as shown in FIG. 6A. Accordingly, the rear support part 3d is moved to a lateral side of the tip portion 44 as shown in FIG. 6B in association with the opening motion of the optical part 2.

The curved surface R is formed under condition that a difference Δd between a distance dl from an axis O of the plunger 40 to the curved surface R and a distance d2 from the axis O to the inner wall surface W of the injection part 20 is sufficiently larger than a diameter Δd2 of the support part 3d (d1 < d2). Accordingly, the gap P is formed between the peripheral surface 44b of the tip portion 44 and the inner wall surface W (near the front end 21a) of the injection part 20 to allow the support part 3d to pass along the outer periphery (the peripheral surface 44b) of the tip portion 44.

Meanwhile, the optical part 2 folded in the injection part 20 is moved in a smaller diameter as approaching the front end 21a of the tapered injection part 20. The optical part 2 thus becomes pushed more strongly by the plunger 40. However, in case the optical part 2 is strongly pushed by the plunger 40 even though the optical part 2 is not sufficiently folded, the soft optical part 2 is deformed, likely causing the tip portion 44 to get onto the optical part 2. Further, a part of the optical part 2 is liable to get into a slight gap occurring between the peripheral surface 44b (the lower surface) of the tip portion 44 and the inner wall surface W of the injection part 20. If the plunger 40 is pushed further ahead while the part of the optical part 2 is getting into the gap, the part of the optical part 2 further slips into the gap. When the optical part 2 in such a state is continuously pushed by the plunger 40, the optical part 2 is more likely to be broken. It is to be noted that when the tip portion 44 gets onto the optical part 2 once, the tip portion 44 also may not appropriately contact with the edge 2b and hence the plunger 40 cannot push out the optical part 2.

In the present embodiment, therefore, the peripheral surface 44b (the lower surface) of the tip portion 44 is formed with the asperities in a range under which the optical part 2 may get into. With such configuration, even when the tip portion 44 is about to get onto the optical part 2 in pushing the IOL 1a (or the IOL 1), the frictional force of the asperities restrains the tip portion 44 from going ahead. This prevents the optical part 2 from getting under the tip portion 44. Herein, an example where a rough surface RF consisting of fine asperities is explained (see FIGs. 6A to 6D and FIG. 7).

In the present embodiment, a maximum height Ry (JIS B 0601 1994) of surface roughness of the asperities (the rough surface RF) is determined as Ry = 6.3 µm, more preferably, between 12.5 µm and 100 µm inclusive. If the maximum height Ry of surface roughness is smaller than 6.3 µm, a sufficient frictional force cannot be obtained, making it difficult to prevent the optical part 2 from getting under the tip portion 44. The maximum height Ry of surface roughness is defined by cutting out a portion stretching over a reference length of an average line from a roughness curve obtained by measurement of the asperities on the surface, measuring a gap between a maximum value (a peak) and a minimum value (a valley) in this portion in a direction of a magnitude axis of the roughness curve, and indicating this value in micrometer.

An average surface roughness Ra of the asperities (the rough surface RF) in the present embodiment is 6.3 µm or more, and more preferably, the average surface roughness Ra is determined between 12.5 µm and 100 µm inclusive. With the average surface roughness Ra determined in this way, the plurality of asperities having a predetermined height can more appropriately prevent the optical part 2 from getting under the tip portion 44.

A method of forming the rough surface RF is explained below. Firstly, an explanation is given to a case where the plunger 40 is made of a metal material such as iron, aluminum, and others. A bulk metal material formed in a predetermined shape is first subjected to a known cutting process to make the outer shape of the plunger 40. Thereafter, the plunger 40 is subjected to a usual mechanical process for metal working such as known shot blasting, cutting, filing with a file having predetermined roughness, and others to form the rough surface RF in a part of the outer surface of the tip portion 44. As an alternative, the bulk material formed in a predetermined shape is subjected to grinding with a sharp blade to form the outer shape of the plunger 40 having a rough surface. Then, the other part of the outer surface of the tip portion 44, other than the range formed with the rough surface RF, is polished into a smooth surface state by a known technique. Alternatively, the entire surface of the plunger 40 may be polished into a smooth surface state once and then a predetermined range is shaved off with a blade, a file, or the like to create the rough surface RF. Furthermore, depending on the material of the plunger 40, it may be arranged to dip the plunger 40 into a specific chemical solution to roughen an entire surface of the plunger 40 and then the surface excepting the range for the rough surface RF is polished into a smooth surface.

On the other hand, in a case where the plunger 40a is made of resin such as plastic, a known molding process is adopted. In this case, asperities are formed in advance in a portion of a (metal) mold desired to create the rough surface RF by shot blasting, etching, and other processing methods. A resin material is then poured in the mold. Consequently, the plunger 40 formed with the rough surface RF is produced. An alternative may be adopted in which the outer shape of the plunger 40 is formed by the metal mold and then the rough surface RF is created in a part of the surface of the plunger 40 by known shot blasting, cutting with a blade, and filing.

In this embodiment, the rough surface RF is formed in the lower surface of the peripheral surface 44b of the tip portion 44, but not limited thereto. The rough surface RF is required only to be formed in a range where the optical part 2 is likely to get into the gap between the tip portion 44 and the inner wall surface W in accordance with the internal shape of the injector 10, the shape of the tip portion 44, or others. In other words, the rough surface RF may be formed in an upper or lower part of the peripheral surface 44b, in both the upper and lower parts, in the entire periphery, or in the entire surface of the plunger 40 excepting the contact surface 44a. Although the rough surface RF is formed in the entire lower surface of the peripheral surface 44b in this embodiment, the rough surface RF has only to be formed in at least a front end portion (close to the contact surface 44a) of the lower surface of the peripheral surface 44b. This makes it possible to prevent a part of the optical part 2 from getting into the gap.

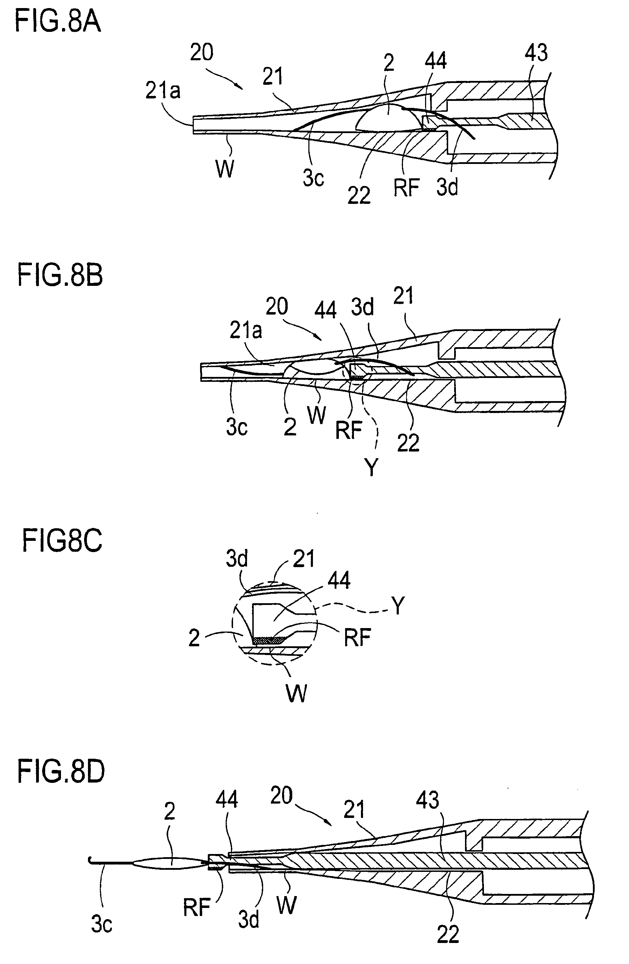

An injecting operation of the IOL using the injector 10 having the above configuration will be explained below. FIGs. 8A to 8D are schematic cross-sectional views of the injection part 20 and its surroundings when viewed from side, in which FIG. 8C is an enlarged view of a part Y in FIG. 8C. The following explanation also exemplifies the case using the three-piece IOL 1a.

Firstly, an operator (a user) puts the IOL 1a with forceps on the setting part 22 in the injection part 20 by opening the lid member 20a, and inserts the front end 21 a into an eye. When the press part 41 of the plunger 40 is pushed from this state toward the front end 21 a, the contact surface 44a comes into contact with the optical part 2 as shown in FIG. 8A. When the press part 41 is pushed further ahead, the IOL 1a is moved into the injection tube 21. Since the opening diameter (inner diameter) of the injection tube 21 is gradually narrower, the optical part 2 is folded (rounded) along the inner wall W of the injection tube 21 so that the front surface 2a is valley folded. On the other hand, the tip portion 44 is moved ahead while the lower surface of the peripheral surface 44b is in rubbing contact with the inner wall surface W. As the optical part 2 is folded, the rear support part 3d is gradually moved upward within the injection part 20.

Because of the gradually narrower opening diameter of the injection part 20, when the optical part 2 is further pushed, a pressing force of the plunger 40 is gradually increased. When the optical part 2 is strongly pushed by the plunger 40, a part of the optical part 2 may get into the gap between the lower surface of the tip portion 44 and the inner wall surface W of the injection part 20. In the present embodiment, however, as shown in FIG. 8B, the frictional force of the rough surface RF formed in the lower surface of the tip portion 44 restrains the optical part 2 from getting under the tip portion 44. FIG. 8C is an enlarged view showing the tip portion 44 and its surroundings in FIG. 8B. It is accordingly possible to prevent breakage of the optical part 2 and also restrain the tip portion 44 from getting onto the optical part 2. Consequently, the entire optical part 2 can appropriately be pushed out.

When the tip portion 44 is moved to the vicinity of the front end 21a of the injection part 20 as mentioned above, the rear support part 3d comes to an upper position in the gap P formed between the tip portion 44 and the inner wall W as shown in FIG. 6A as the optical part 2 is folded. When the plunger 40 is pushed further ahead from this state, the optical part 2 is first injected into the eye through the front end 21 a as shown in FIG. 8D. At that time, the rear support part 3d is left in the injection part 20. As the optical part 2 is opened, the rear support part 3d is moved to escape from above the tip portion 44 to the lateral side through the gap P as shown in FIG. 6B. This prevents the rear support part 3d from becoming caught between the plunger 40 and the front end 21a of the injection part 20 during the opening motion of the optical part 2. Defects such as breakage of the support part 3d are thus avoided, so that the optical part 2 is allowed to stably and appropriately open in the eye. Thereafter, when the rear support part 3d is pushed into the eye, the support part 3d is restored to its original shape in the eye by the restoring force and then is positioned along a capsule by stress applied to the support part 3d from the interior of the eye. The optical part 2 is therefore appropriately held in the eye by the support parts 3 (3c and 3d).

As above, the gap P is provided to allow the rear support part 3d to move inside the front end 21a along the outer periphery of the tip portion 44 while the tip portion 44 of the plunger 40 is placed near the front end 21a of the injection part 20. This allows the IOL 1a to appropriately open in the eye.

The present invention is not limited to the above configuration. For instance, the shape of the tip portion 44 for forming the gap P may be not only the curved surface but also another shape. For example, it may be a flat surface formed by cutting as shown in FIG. 6C or a recessed or stepped surface as shown in FIG. 6D. In addition to those mentioned above, the shape of the tip portion 44 may be any shape capable of forming the gap P to movably place the support part 3d in association with the opening motion of the optical part 2 while the tip portion 44 is in the vicinity of the front end 21 a.

To more appropriately open the IOL 1 in the eye by avoiding the support part 3d from becoming caught, the peripheral surface 44b (the upper surface) of the tip portion 44 is preferably formed as a slant surface SL as shown in FIG. 7. The slant surface SL of the tip portion 44 is formed sloped so that its cross sectional area taken perpendicular to the push-out direction gradually decreases from the contact surface 44a toward the push rod 43. With such configuration, the gap P in which the support part 3d is movably placed is made wider as the plunger 40 is pushed ahead from a state where the tip portion 44 is located near the front end 21 a. Thus, the support part 3d is easy to return to its original state in association with the push-out operation from the injection part 20. This configuration makes it possible to further reduce the influence of the support part 3d left in the injection part 20 on the optical part 2.

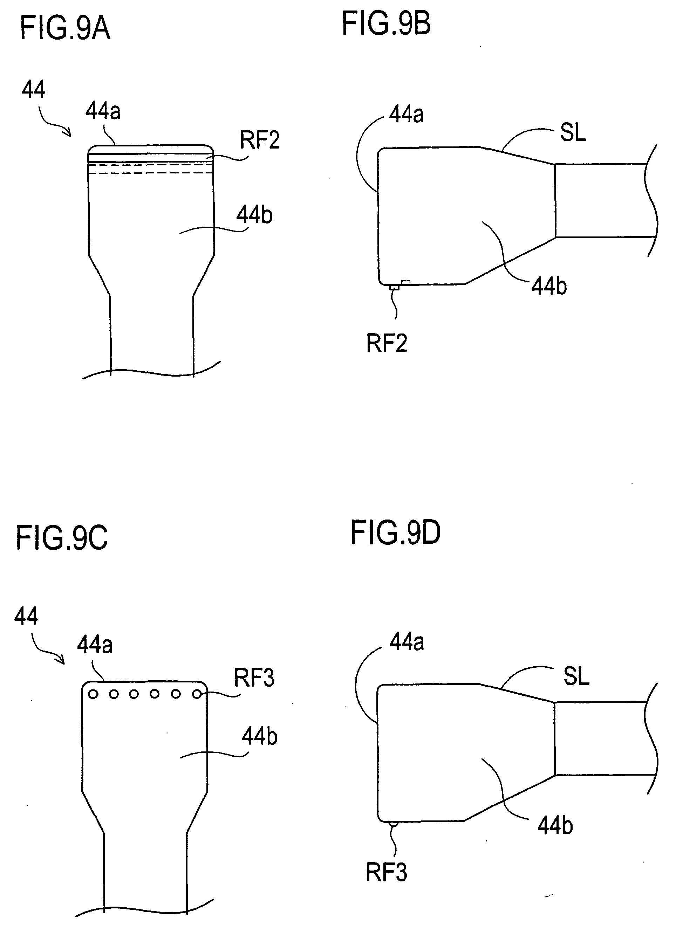

In the above mentioned, the asperities formed in the tip portion 44 are exemplified as the rough surface RF formed by roughening the surface by abrasion with a blade or a file, but not limited thereto. For example, the asperities may be selected from various kinds of modified patterns shown in FIGs. 9A to 9D. Specifically, FIGs. 9A and 9B describe a first modified example and FIGs. 9C and 9D describe a second modified example. FIG. 9A is a bottom view of a tip portion 44 of the first modified example viewed from below and FIG. 9B is a side view of the tip portion 44 of the first modified example. FIG. 9C is a bottom view of a tip portion 44 of the second modified example seen from below and FIG. 9D is a side view of the tip portion 44 of the second modified example.

As shown in FIG. 9A, for example, the asperities may be formed as a protrusion or recess (herein, referred to as a rough surface RF2) formed linearly extending in a direction perpendicular to the push-out direction. In this case, the rough surface RF2 is made by cutting or molding. It is to be noted that the linear protrusion or recess forming the rough surface RF2 is required only to be formed in at least an end part of the tip portion 44 near the contact surface 44a. As an alternative, the rough surface RF2 may be formed of linear protrusions and linear recesses arranged alternately.

As shown in FIG. 9C, as another alternative, the asperities may be formed of a combination of dots (herein, referred to as a rough surface RF3). Such rough surface RF3 is made by molding. In this case, the rough surface RF3 may also be formed in at least a part of the tip portion 44 near the contact surface 44a, under which the optical part 2 is liable to get into. The maximum height Ry of each surface roughness of the rough surface RF2 and the rough surface RF3 shown in FIGs. 9A to 9D is determined as Ry = 6.3 µm or more and, more preferably, between 50 µm and 200 µm inclusive.

The above explanation exemplifies the structure of an injector configured to valley-fold the IOL 1a. Depending on the type of injector, the optical part 2 is mountain-folded. In this type, a fold position (line) of the optical part 2 is on an upper side in the insertion part 20, and the fold position is pushed by the tip portion 44. In this case, specifically, the rear support part 3d is located below the tip portion 44 while the IOL 1a is in a folded state in the injection tube 21. Accordingly, in this case, a gap P is formed to allow the support part 3d to escape into between the lower surface of the tip portion 44 and the lateral side surface continuous with the lower surface. In other words, the tip portion 44 is provided with a curved surface, a slant surface, or a recessed surface from the bottom to the lateral side.

In this case, as mentioned above, the rough surface RF is formed in at least a predetermined region (an upper surface) of the peripheral surface 44b of the tip portion 44. Accordingly, it is possible to restrain the optical part 2 from getting into a gap between the tip portion 44 and the inner wall of the injection part 20, thus preventing the optical part 2 from getting broken and the tip portion 44 from getting under the optical part 2. Consequently, the IOL 1a is appropriately pushed out.

Furthermore, depending on the structure of injector, the fold position of the optical part 2 is located on a side (left or right) region in the injection part 20. In this case, the asperities (the rough surface RF and others) may be formed in at least a right or left surface of the peripheral surface 44b of the tip portion 44 according to the fold position of the optical part 2. For instance, in the case where the optical part 2 is valley-folded on the left side in the injection part 20, the asperities are formed on the left surface of the peripheral surface 44b of the tip portion 44. Specifically, the asperities (the rough surface RF and others) is formed in such a position (a predetermined region of the outer periphery of the tip portion 44) as to generate a predetermined frictional force in a contact position of the tip portion 44 with respect to the optical part 2, thereby preventing a part of the optical part 2 from getting into between the tip portion 44 and the inner wall surface W.

The above explanation shows the example that the asperities are formed in the tip portion 44 of the plunger 40 but the present invention is not limited thereto. In the conventional structure, after the optical part pushed by the plunger is placed in the eye, the rear support part may be left in the injection part. In this case, an operator has to repeat a push-out operation using the injector to push the rear support part into the eye. Therefore, an injector intended for a one-piece IOL has been proposed in which a groove is formed in a tip portion of a plunger to receive a rear support part in pushing the IOL, thus enabling push-out of the entire IOL (EP 2177178 A1, JP 2009-18009A). In this type of injector, however, the shape of the tip portion of the plunger is complicated. In the second embodiment, therefore, asperities (herein, referred to as a rough surface RF4) are formed in a part of the outer surface of the plunger 40 (the shaft 45) to increase friction with the rear support part 3b.

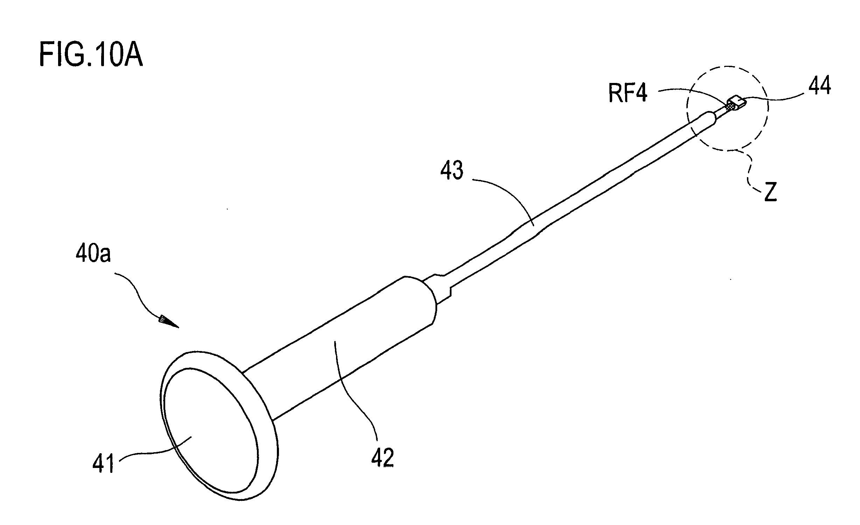

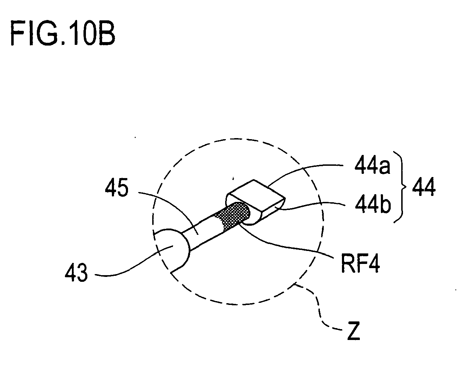

Herein, the structure of a plunger 40a in a second embodiment will be explained referring to FIGs. 10A and 10B. Specifically, FIG. 10A is a perspective view of the plunger 40a and FIG. 10B is an enlarged view of a tip portion 44 and its surroundings in the plunger 40a indicated by a part Z in FIG. 10A. In the following explanation, like parts to those of the aforementioned injector 10 are explained with the same reference signs. The following explanation shows an example using a one-piece IOL.

The plunger 40a in this embodiment is formed with the asperities (the rough surface RF4) in a part of the outer surface (the shaft 45) to increase the frictional force with the rear support part 3b.

FIGs. 11A and 11B are enlarged views of the tip portion 44 and its surroundings of the plunger 40a. Specifically, FIG. 11A is a side view of the tip portion 44 and its surroundings and FIG. 11B is a top view of the tip portion 44 and its surroundings. In this embodiment, concretely, the rough surface RF4 is formed on the entire periphery of a part (the surface in a predetermined range from a connecting position with the tip portion 44) of the shaft 45 placed in a range (a contact region) which will contact with the rear support part 3b during push-out of the IOL 1. Since the rough surface RF4 is formed to increase friction between the plunger 40a and the support part 3b, the support part 3b is held by the friction of the rough surface RF4 during push-out of the IOL 1. Accordingly, the rear support part 3b is prevented from sliding on the surface of the plunger 40a and thus spreading backward in the forward moving direction of the IOL 1. This reduces defects such as breakage of the support part 3b resulting from twining thereof around the plunger 40a. By use of a simply configured plunger, the entire IOL 1 including the support part 3b is allowed to be pushed at once toward the front end 21a.

The asperities (the rough surface RF4 and others) shown in the present embodiment are provided in the form of a number of protrusions and recesses or a number of grooves on the surface. The degree (surface roughness) thereof is defined by the maximum height Ry of surface roughness presented in the aforementioned "JIS B 0601 1994" and the details of the maximum height Ry of surface roughness are omitted here.

For instance, the maximum height Ry of the rough surface RF4 in this embodiment is determined to be 6.3 µm or more and more preferably to 10 µm or more. If the maximum height Ry of the rough surface RF4 is lower than 6.3 µm, the friction between the rough surface RF and the support part 3b is smaller than the friction between the inner wall surface W of the injection tube 21 and the support part 3b. It is therefore hard for the rough surface RF to hold the support part 3b. It is to be noted that the maximum height Ry needs only be large enough to allow the rough surface RF to hold the support part 3b and also cause a frictional force allowing forward movement of the support part 3b in association with forward movement of the plunger 40a. The range of the maximum height Ry of surface roughness indicated in this embodiment is a mere example and can be determined to meet the above conditions in consideration of a combination of the kind of material of the IOL 1 and the kind of material of the plunger 40a.

An average surface roughness Ra of the rough surface RF4 is determined to be 6.3 µm or more and more preferably 10 µm or more. With such configuration, the rough surface RF4 is formed of the plurality of asperities having the predetermined maximum height Ry. Accordingly, a part of the support part 3b is appropriately held by the rough surface RF4.

A method of forming the plunger 40a having the above rough surface RF4 is the same as the aforementioned processing method of the plunger 40 and the details thereof are not repeated here.

This embodiment shows an example where the asperities (the rough surface RF4 and others) are formed in the entire periphery of a part of the shaft 45 in which the support part 3b is placed. However, the present invention is not limited thereto. The asperities (the rough surface RF4 and others) need only be formed so as to include the range of the plunger 40a with which the support part 3b comes into contact according to the internal shape of the injection part 20 the shape of the tip portion 44 of the plunger 40a.

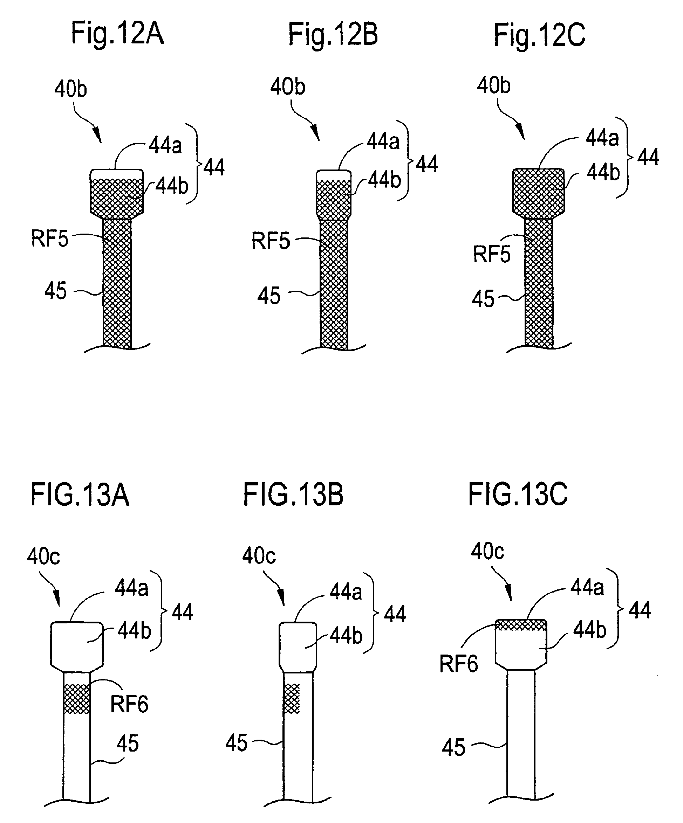

FIGs. 12A to 12C show a structure of a plunger 40b of a modified example. Specifically, FIG. 12A is a top view of the plunger 40b (a tip portion 44 side), FIG. 12B is a side view of the plunger 40b, and FIG. 12C is a bottom view of the plunger 40. As shown in the figures, asperities (herein, referred to as a rough surface RF5) can also be formed in an entire surface of the plunger 40b so as to include a range of the plunger 40b with which the support part 3b will come into contact. In this case, however, the rough surface RF5 is formed in a range excepting a contact surface 44a which will contact with the optical part 2 and a part of the plunger 40b (the tip portion 44) which a part of the optical part 2 may get onto when the optical part 2 is pushed by the plunger 40b. For example, the range is defined as a range corresponding to a gap generated between the inner wall surface W of the injection tube 21 and the peripheral surface of the tip portion 44, but not including a portion on the contact surface 44a side. Accordingly, during push-out of the IOL 1, the support part 3b is held by the frictional force of the rough surface RF5, and the rough surface RF5 is prevented as much as possible from coming contact with the optical part 2. Consequently, the IOL 1 can appropriately be pushed into the eye.

As shown in FIG. 12C, the lower surface (at least on the contact surface 44a side) of the peripheral surface 44b of the tip portion 44 is formed with the asperities (the rough surface RF5 and others). As mentioned above, it is therefore possible to minimize the possibility that the optical part 2 gets into a gap between the inner wall surface W of the injection tube 21 and the tip portion 44 of the plunger 40b during a push-out operation of the IOL 1. This can appropriately push out the optical part 2.

FIGs. 13A to 13C show a structure of a plunger 40c of another modified example. Specifically, FIG. 13A is a top view of the plunger 40c, FIG. 13B is a side view of the plunger 40c, and FIG. 13C is a bottom view of the plunger 40c. As above, asperities (herein, referred to as a rough surface RF6) to hold the support part 3b may be formed in only at least an upper surface of the plunger 40c with which the support part 3b comes into contact. In this case, furthermore, as shown in FIG. 13C, the rough surface RF6 is formed in the lower surface (at least a portion near the contact surface 44a) of the peripheral surface 44b of the tip portion 44. This can prevent the optical part 2 from getting into a gap between the inner wall surface W of the injection part 20 and the tip portion 44 of the plunger 40c. The asperities to prevent the optical part 2 from entering the gap are formed according to a folding direction of the optical part 2. The aforementioned rough surface RF2, rough surface RF3, and others may be combined.

Besides the above mentioned, in an outer peripheral region of a plunger excepting the contact surface 44a, the rough surface has only to be formed on a part or entire region of at least one of an upper surface, a lower surface, or a side surface of the plunger with which the rear support part may contact during push-out of the IOL.

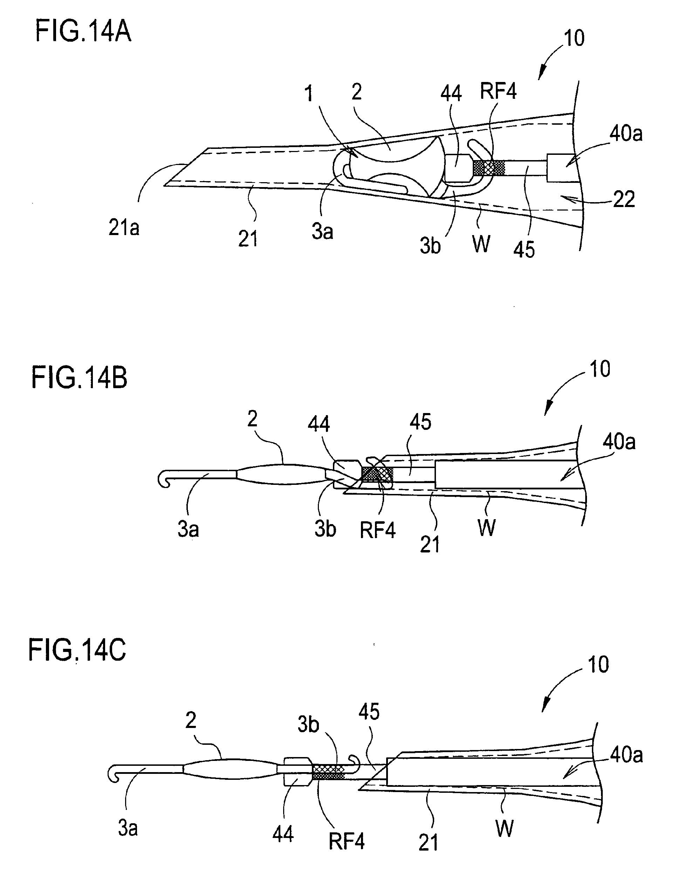

The following explanation is given to a push-out operation of the IOL 1 using the injector 10 (the plunger 40a) in the second embodiment having the above configuration. FIGs. 14A to 14C are explanatory views showing the push-out operation of the IOL 1. An operator (a user) puts the IOL 1 with forceps onto the setting part 22 in the injection part 20 by opening the lid member 20a, and inserts the front end 21a into an eye. When the press part 41 is pushed from this state, the support part 3b comes into contact with the surface of the plunger 40a. When the press part 41 is pushed further ahead, the support part 3b comes into contact with the rough surface RF4 as shown in FIG. 14A. The support part 3b is thus held by a frictional force of the rough surface RF4. On the other hand, when the plunger 40a is pushed toward the front end 21 a, the contact surface 44a comes into contact with the optical part 2. When the plunger 40a is further pushed from this state, the optical part 2 is gradually folded (rounded) along the inner wall surface W of the injection part 20. At that time, the support part 3b held by the rough surface RF4 as shown in FIG. 14A is also moved toward the front end 21a in association with the push-out of the plunger 40a. Accordingly, the support part 3b is prevented from sliding on the surface of the plunger 40a and spreading backward in the forward moving direction of the IOL, so that defects such as breakage of the support part 3b resulting from twining thereof around the plunger 40b.

As above, the optical part 2 injected out of the front end 21a is gradually opened in a capsule as shown in FIG. 14B. At that time, since the rear support part 3b is held by the rough surface RF4, the entire IOL 1 is prevented from abruptly separating from the front end 21a. Accordingly, the optical part 2 can be stably opened in the capsule. Further, the optical part 2 is opened by centering around the support part 3b held by the rough surface RF4, so that the opening direction of the IOL 1 is determined as one direction.

When the plunger 40a is further pushed until the rough surface RF4 holding the support part 3b comes out of the front end 21a as shown in FIG. 14C, the support part 3b is separated from the rough surface RF4 by a restoring force of the support part 3b. By the stress applied to the optical part 2 from the interior of the eye, the optical part 2 is then positioned along the capsule. Accordingly, the optical part 2 is appropriately held by the support parts 3 in the eye.

Since the rough surface is formed in the outer peripheral surface of the plunger as mentioned above, the rear support part is held by the frictional force of the rough surface. Thus, the rear support part is moved as the plunger is moved ahead. This prevents the rear support part from being left in the injection part and thus avoiding defects such as breakage resulting therefrom. By use of the plunger simply configured as above, it is possible to easily push out the IOL into the eye appropriately with one push operation.

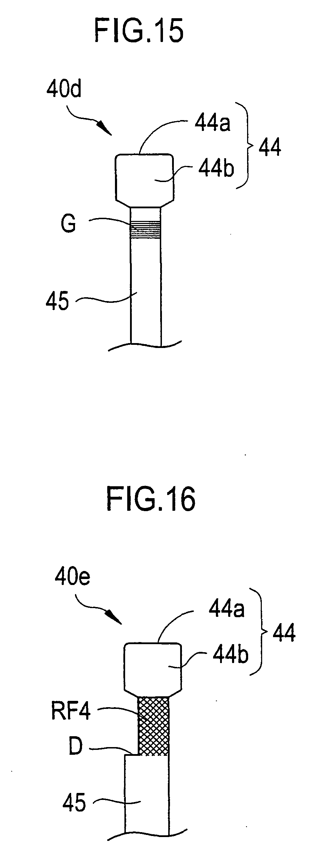

In the above explanation, the asperities forming the rough surface RF4 are provided in a part of the outer surface of the plunger 40a in order to generate the frictional force to hold the support part 3b. However, the present invention is not limited thereto. A plunger 40d of a modified example is shown in FIG. 15, which is a top view. In this case, asperities are provided in the form of grooves G in the surface of the plunger 40d in a range in which the support part 3b will come into contact with the plunger 40d, to increase a frictional force with respect to the support part 3b. In the case where the plunger 40d is made of metal, the grooves G are formed by known milling, lathe working, or abrading (grinding). On the other hand, in the case where the plunger 40d is made of resin, a metal mold is designed in advance to have asperities corresponding to the grooves G as mentioned above.

FIG. 16 is an explanatory view showing a structure of a plunger 40e of another modified example. As shown in FIG. 16, a contact surface D may be formed in the plunger 40e near a tip portion in order to push the rear support part 3b (or the support part 3d). The contact surface D is located in a position where a part of the support part 3b appropriately comes into contact therewith (i.e., in a position where no failures occur) during push-out of the IOL 1. With such configuration, when the optical part 2 is pushed by the tip portion 44 (the contact surface 44a), the support part 3b (or the support part 3d) is pushed by the contact surface D. Thus, the IOL 1 can be efficiently pushed into the eye.

Although the above second embodiment shows the example using the one-piece IOL, depending on the kind of IOL, i.e., even when the IOL is a three-piece IOL, the simply configured injector including the plunger having the aforementioned structure according to the present invention can hold the support part during push-out of the IOL.

Since the asperities are formed in a part of the outer peripheral region of the plunger as mentioned above, a larger frictional force is generated in a contact portion of the asperities of the plunger with respect to the IOL than a frictional force generated in the other part of the outer peripheral region of the plunger formed with no asperities. It is consequently possible to appropriately prevent the occurrence of defects of the IOL such as breakage of a part of the IOL when pushed out.

While the presently preferred embodiment of the present invention has been shown and described, it is to be understood that this disclosure is for the purpose of illustration and that various changes and modifications may be made without departing from the scope of the invention as set forth in the appended claims.

## Claims

1. An intraocular lens injection instrument (10) for injecting an 10L (1, 1a) including an optical part (2) and support parts (3) into an eye by folding the IOL, comprising:
a cylindrical body part (30) provided, at its front end, with an injection tubular part (20) configured to inject the IOL (1,1a) into the eye through an incision formed in the eye and including a setting part (22) in which the IOL (1,1a) is to be set; and
a plunger (40, 40b-40e) for pushing the IOL (1,1a) out of the injection tubular part (20), the plunger (40,40b-40c) being placed in the cylinder body part (30) to be movable in an axial direction of the cylinder body part (30) and having a tip portion (44),
**characterized by**
a part of an outer peripheral region (44b,45) of the plunger (40,40b-40e) to which the IOL (1,1a) comes into contact when the IOL is to be pushed ahead is formed with asperities (RF, RF2-RF6, G) to generate a predetermined frictional force by contact with the IOL (1,1a), the predetermined frictional force being larger than a frictional force generated in the other part of the outer peripheral region of the plunger (40,40b-40e) by contact with the IOL (1,1a).

2. The IOL injection instrument (10) according to claim 1, wherein the tip portion (44) of the plunger (40,40b-40e) is formed with the outer peripheral region (44b) a part of which has an outer peripheral shape along an inner wall (W) of the injection tubular part (20), and
the asperities (RF,RF2-RF6,G) are formed in at least a predetermined region of the part of the outer peripheral region (44b).

3. The IOL injection instrument (10) according to claim 2, wherein the predetermined frictional force of the asperities (RF,RF 2-RF6,G) formed in the predetermined region is large enough to prevent a part of the optical part (2) of the IOL (1,1a) from getting into a gap between the tip portion (44) of the plunger (40,40b-40) and the inner wall of the injection tubular part (20) when the plunger (40,40b-40e) is moved ahead.

4. The IOL injection instrument (10) according to claim 2 or 3, wherein the predetermined region is at least one of an upper surface and a lower surface of the tip portion (44) of the plunger (40,40b-40e).

5. The IOL injection instrument (10) according to one of claims 1 to 4, wherein the asperities (RF,RF2-RF6,G) are formed in at least a part of a contact region of the plunger as the part of the outer peripheral region (45) with which one of the support parts (3) of the IOL (1,1a) comes into contact in order to generate the predetermined frictional force between the plunger (40,40b-40e) and the support part (3) of the IOL (1,1a) during push-out of the IOL (1,1a) placed on the setting part (22).

6. The IOL injection instrument (10) according to claim 5, wherein the predetermined frictional force of the asperities (RF,RF2-RF6,G) formed in the contact region is large enough to allow the support part (3) of the IOL (1,10) in contact with the contact region to move ahead, without sliding on the plunger (40,40b-40e), in association with forward movement of the plunger (40,40b-40e).

7. The IOL injection instrument (10) according to one of claims 1 to 6, wherein the asperities (RF,RF2-RF6,G) are a rough surface.

8. The IOL injection instrument (10) according to claim 7, wherein a maximum height (Ry) of surface roughness of the rough surface is 6.3 µm or more.

9. The IOL injection instrument (10) according to claim 8, wherein
a gap (P) is formed between the tip portion (44) of the plunger (40,40b-40e) and a front end (21a) of the injection tubular part (20) to allow passage of a rear one (3b, 3d) of the support parts of the IOL (1,1a) along an outer periphery (44b) of the tip portion (44) of the plunger (40,40b-40e), the gap being provided to allow the rear support part (3b,3d) of the IOL (1,1a) to escape along the outer periphery (44b) of the tip portion (44) of the plunger (40,40b-40e) when the rear support part (3b,3d) is moved in association with a folding motion of the IOL (1,1a) in the injection tubular part (20) and an opening motion of the IOL (1,1a) injected out of the front end of the injection tubular part (20).

10. The IOL injection instrument (10) according to claim 9, wherein
the gap is formed by a shape of at least a predetermined region of the part of the outer peripheral region (44b) of the tip portion (44) of the plunger (40,40b-40e) the shape having a curve, slant, or recessed surface so that at least the predetermined region is apart from the inner wall (W) of the injection tubular part (20) to allow passage of at least the rear support part (3b,3b) of the IOL (1,1a).

11. The IOL injection instrument according to claim 10, wherein the predetermined region is a region from an upper surface to a side surface or from a lower surface to the side surface of the tip portion (44) of the plunger (40,40b-40e).

## Patentansprüche

1. Intraokularlinsen-Injektionsinstrument (10) zum Injizieren einer IOL (1, 1a) mit einem optischen Teil (2) und Stützteilen (3) in ein Auge durch Falten der IOL, aufweisend:
einen zylindrischen Körper (30), der an seinem vorderen Ende mit einer Injektionsröhre (20) versehen ist, die ausgestaltet ist, um die IOL (1, 1a) durch einen im Auge ausgebildeten Schnitt in das Auge zu injizieren, und die ein Einsetzteil (22) enthält, in das die IOL (1, 1a) einzusetzen ist; und
einen Kolben (40, 40b-40e) zum Drücken der IOL (1, 1a) aus der Injektionsröhre (20), wobei der Kolben (40, 40b-40e) so in dem zylindrischen Körper (30) angeordnet ist, dass er in einer axialen Richtung des zylindrischen Körpers (30) bewegbar ist, und einen Spitzenabschnitt (44) aufweist,
**dadurch gekennzeichnet, dass**
ein Teil eines Außenumfangsbereichs (44b, 45) des Kolbens (40, 40b-40e), mit dem die IOL in Kontakt kommt, wenn die IOL nach vorne gedrückt wird, mit Oberflächenunebenheiten (RF, RF2-RF6, G) ausgebildet ist, um durch Kontakt mit der IOL (1, 1a) eine vorbestimmte Reibungskraft zu erzeugen, wobei die vorbestimmte Reibungskraft größer ist als eine Reibungskraft, die in dem anderen Teil des Außenumfangsbereichs des Kolbens (40, 40b-40e) durch Kontakt mit der IOL (1, 1a) erzeugt wird.

2. IOL-Injektionsinstrument (10) nach Anspruch 1, bei welchem
der Spitzenabschnitt (44) des Kolbens (40, 40b-40e) mit dem Außenumfangsbereich (44b) ausgebildet ist, von dem ein Teil eine Außenumfangsform entlang einer Innenwand (W) der Injektionsröhre (20) hat, und
die Oberflächenunebenheiten (RF, RF2-RF6, G) in wenigstens einem vorbestimmten Bereich des Teils des Außenumfangsbereichs (44b) ausgebildet sind.

3. IOL-Injektionsinstrument (10) nach Anspruch 2, bei welchem
die vorbestimmte Reibungskraft der in dem vorbestimmten Bereich ausgebildeten Oberflächenunebenheiten (RF, RF2-RF6, G) groß genug ist, um zu verhindern, dass ein Teil des optischen Teils (2) der IOL (1, 1a) in einen Spalt zwischen dem Spitzenabschnitt (44) des Kolbens (40, 40b-40e) und der Innenwand der Injektionsröhre (20) gelangt, wenn der Kolben (40, 40b-40e) nach vorne bewegt wird.

4. IOL-Injektionsinstrument (10) nach Anspruch 2 oder 3, bei welchem
der vorbestimmte Bereich wenigstens eine einer oberen Oberfläche und einer unteren Oberfläche des Spitzenabschnitts (44) des Kolbens (40, 40b-40e) ist.

5. IOL-Injektionsinstrument (10) nach einem der Ansprüche 1 bis 4, bei welchem
die Oberflächenunebenheiten (RF, RF2-RF6, G) in wenigstens einem Teil eines Kontaktbereichs des Kolbens als der Teil des Außenumfangsbereichs (45), mit dem eines der Stützteile (3) der IOL (1, 1a) in Kontakt kommt, ausgebildet sind, um während des Herausdrückens der in dem Einsetzteil (22) platzierten IOL (1, 1a) die vorbestimmte Reibungskraft zwischen dem Kolben (40, 40b-40e) und dem Stützteil (3) der IOL (1, 1a) zu erzeugen.

6. IOL-Injektionsinstrument (10) nach Anspruch 5, bei welchem
die vorbestimmte Reibungskraft der in dem Kontaktbereich ausgebildeten Oberflächenunebenheiten (RF, RF2-RF6, G) groß genug ist, um das Stützteil (3) der IOL (1, 1a) in Kontakt mit dem Kontaktbereich zusammen mit der Vorwärtsbewegung des Kolbens (40, 40b-40e) ohne Gleiten am Kolben (40, 40b-40e) nach vorne bewegen zu lassen.

7. IOL-Injektionsinstrument (10) nach einem der Ansprüche 1 bis 6, bei welchem
die Oberflächenunebenheiten (RF, RF2-RF6, G) eine rauhe Oberfläche sind.

8. IOL-Injektionsinstrument (10) nach Anspruch 7, bei welchem
eine maximale Höhe (Ry) einer Oberflächenrauheit der rauhen Oberfläche 6,3 µm oder mehr beträgt.

9. IOL-Injektionsinstrument (10) nach Anspruch 8, bei welchem
ein Spalt (P) zwischen dem Spitzenabschnitt (44) des Kolbens (40, 40b-40e) und einem vorderen Ende (21a) der Injektionsröhre (20) ausgebildet ist, um einen Durchgang eines hinteren (3b, 3d) der Stützteile der IOL (1, 1a) entlang eines Außenumfangs (44b) des Spitzenabschnitts (44) des Kolbens (40, 40b-40e) zu erlauben, wobei der Spalt so vorgesehen ist, dass er das hintere Stützteil (3b, 3d) der IOL (1, 1a) entlang des Außenumfangs (44b) des Spitzenabschnitts (44) des Kolbens (40, 40b-40e) entweichen lässt, wenn das hintere Stützteil (3b, 3d) zusammen mit einer Faltbewegung der IOL (1, 1a) in der Injektionsröhre (20) und einer Öffnungsbewegung der aus dem vorderen Ende der Injektionsröhre (20) heraus injizierten IOL (1, 1a) bewegt wird.

10. IOL-Injektionsinstrument (10) nach Anspruch 9, bei welchem
der Spalt durch eine Form wenigstens eines vorbestimmten Bereichs des Teils des Außenumfangsbereichs (44b) des Spitzenabschnitts (44) des Kolbens (40, 40b-40e) gebildet ist, wobei die Form eine Krümmung, eine Schräge oder eine vertiefte Oberfläche aufweist, sodass wenigstes der vorbestimmte Bereich von der Innenwand (W) der Injektionsröhre (20) entfernt ist, um einen Durchgang wenigstens des hinteren Stützteils (3b, 3d) der IOL (1, 1a) zu ermöglichen.

11. IOL-Injektionsinstrument (10) nach Anspruch 10, bei welchem
der vorbestimmte Bereich ein Bereich von einer oberen Oberfläche zu einer Seitenfläche oder von einer unteren Oberfläche zur Seitenfläche des Spitzenabschnitts (44) des Kolbens (40, 40b-40e) ist.

## Revendications

1. Instrument d'injection de lentille intraoculaire (10) destiné à injecter une lentille intraoculaire (IOL) (1, 1a), comportant une partie optique (2) et des parties de support (3) dans un oeil, en repliant la lentille intraoculaire, comprenant :
une partie de corps cylindrique (30) comportant, sur son extrémité avant, une partie tubulaire d'injection (20) configurée de manière à injecter la lentille intraoculaire (1, 1a) dans l'oeil à travers une incision formée sur l'oeil et comportant une partie de mise en place (22) dans laquelle la lentille intraoculaire (1, 1a) doit être placée ; et
un piston (40, 40b à 40e) destiné à pousser la lentille intraoculaire (1, 1a) hors de la partie tubulaire d'injection (20), le piston (40, 40b à 40e) étant placé sur la partie de corps de cylindre (30) de manière à pouvoir être déplacé dans une direction axiale de la partie de corps cylindrique (30) et présentant une partie d'extrémité (44),
**caractérisé par**
une partie d'une zone périphérique externe (44b, 45) du piston (40, 40b à 40e), sur laquelle la lentille intraoculaire (1, 1a) vient en contact lorsque la lentille intraoculaire doit être poussée vers l'avant, comporte des aspérités (RF, RF2 à RF6, G) destinées à produire un effort de frottement prédéterminé par contact avec la lentille intraoculaire (1, 1a), l'effort de frottement prédéterminé étant supérieur à un effort de frottement produit sur l'autre partie de la zone périphérique externe du piston (40, 40b à 40e) par contact avec la lentille intraoculaire (1, 1a).

2. Instrument d'injection de lentille intraoculaire (10) selon la revendication 1, dans lequel la partie d'extrémité (44) du piston (40, 40b à 40e) comporte la zone périphérique externe (44b) dont une partie présente une forme périphérique externe suivant une paroi interne (W) de la partie tubulaire d'injection (20), et
les aspérités (RF, RF2 à RF6, G) sont formées sur au moins une zone prédéterminée de la partie de la zone périphérique externe (44b).

3. Instrument d'injection de lentille intraoculaire (10) selon la revendication 2, dans lequel l'effort de frottement prédéterminé des aspérités (RF, RF2 à RF6, G) formées dans la zone prédéterminée est suffisamment important pour empêcher qu'une partie de la partie optique (2) de la lentille intraoculaire (1, 1a) n'entre dans un jeu entre la partie d'extrémité (44) du piston (40, 40b à 40e) et la paroi interne de la partie tubulaire d'injection (20) lorsque le piston (40, 40b à 40e) est déplacé vers l'avant.

4. Instrument d'injection de lentille intraoculaire (10) selon la revendication 2 ou 3, dans lequel la zone prédéterminée est au moins l'une d'une surface supérieure et d'une surface inférieure de la partie d'extrémité (44) du piston (40, 40b à 40e).

5. Instrument d'injection de lentille intraoculaire (10) selon l'une des revendications 1 à 4, dans lequel les aspérités (RF, RF2 à RF6, G) sont formées sur au moins une partie d'une zone de contact du piston comme la partie de la zone périphérique externe (45) avec laquelle l'une des parties de support (3) de la lentille intraoculaire (1, 1a) vient en contact dans le but de produire l'effort de frottement prédéterminé entre le piston (40, 40b à 40e) et la partie de support (3) de la lentille intraoculaire (1, 1a) au cours de l'éjection de la lentille intraoculaire (1, 1a) placée sur la partie de mise en place (22).

6. Instrument d'injection de lentille intraoculaire (10) selon la revendication 5, dans lequel l'effort de frottement prédéterminé des aspérités (RF, RF2 à RF6, G) formées sur la zone de contact est suffisamment important pour permettre que la partie de support (3) de la lentille intraoculaire (1, 1a) en contact avec la zone de contact se déplace vers l'avant, sans glisser sur le piston (40, 40b à 40e), en association avec le mouvement vers l'avant du piston (40, 40b à 40e).

7. Instrument d'injection de lentille intraoculaire (10) selon l'une des revendications 1 à 6, dans lequel les aspérités (RF, RF2 à RF6, G) sont une surface rugueuse.

8. Instrument d'injection de lentille intraoculaire (10) selon la revendication 7, dans lequel une hauteur maximum (Ry) de la rugosité de surface de la surface rugueuse est supérieure ou égale à 6,3 µm.

9. Instrument d'injection de lentille intraoculaire (10) selon la revendication 8, dans lequel
un jeu (P) est formé entre la partie d'extrémité (44) du piston (40, 40b à 40e) et une extrémité avant (21a) de la partie tubulaire d'injection (20) afin de permettre le passage d'une partie arrière (3b, 3d) des parties de support de la lentille intraoculaire (1, 1a) le long d'une périphérie externe (44b) de la partie d'extrémité (44) du piston (40, 40b à 40e), le jeu étant formé afin de permettre le déplacement de la partie de support arrière (3b, 3d) de la lentille intraoculaire (1, 1a) le long de la périphérie externe (44b) de la partie d'extrémité (44) du piston (40, 40b à 40e) lorsque la partie de support arrière (3b, 3d) est déplacée en association avec un mouvement de pliage de la lentille intraoculaire (1, 1a) dans la partie tubulaire d'injection (20), et un déplacement d'ouverture de la lentille intraoculaire (1, 1a) injectée par l'extrémité avant de la partie tubulaire d'injection (20).

10. Instrument d'injection de lentille intraoculaire (10) selon la revendication 9, dans lequel
le jeu est formé par une forme d'au moins une zone prédéterminée de la partie de la zone périphérique externe (44b) de la partie d'extrémité (44) du piston (40, 40b à 40e), la forme comprenant une courbe, un plan incliné ou une surface creuse de telle sorte qu'au moins la zone prédéterminée est séparée de la paroi interne (W) de la partie tubulaire d'injection (20) afin de permettre le passage d'au moins la partie de support arrière (3b, 3d) de la lentille intraoculaire (1, 1a).

11. Instrument d'injection de lentille intraoculaire selon la revendication 10, dans lequel la zone prédéterminée est une zone à partir d'une surface supérieure vers une surface latérale ou à partir d'une surface inférieure vers la surface latérale de la partie d'extrémité (44) du piston (40, 40b à 40e).
